(19) Europäisches Patentamt
European Patent Office
Office européen des brevets

(11) Publication number: **0 610 032 A1**

(12) **EUROPEAN PATENT APPLICATION**

(21) Application number: **94300629.6**

(22) Date of filing: **27.01.94**

(51) Int. Cl.$^5$: **C07D 409/04,** C07D 417/04, C07D 409/14, A61K 31/38

(30) Priority: **05.02.93 EP 93400300**

(43) Date of publication of application: **10.08.94 Bulletin 94/32**

(84) Designated Contracting States: **AT BE CH DE DK ES FR GB GR IE IT LI LU MC NL PT SE**

(71) Applicant: **ZENECA LIMITED**
**Imperial Chemical House, 9 Millbank**
**London SW1P 3JF (GB)**

(71) Applicant: **ZENECA-PHARMA**
**Immeuble "Le Galien",**
**1, Rue des Chauffours,**
**B.P. 127**
**F-95022 Cergy Pontoise Cédex (FR)**

(72) Inventor: **Crawley, Graham Charles**
**Zeneca Pharmaceuticals,**
**Alderley Park**
**Macclesfield, Cheshire, SK10 4TG (GB)**

(74) Representative: **Tait, Brian Steele et al**
**ICI Group Patents Service Dept.**
**PO Box 6**
**Shire Park**
**Bessemer Road**
**Welwyn Garden City Herts AL7 1 HD (GB)**

(54) **Acetanilide derivatives.**

(57) The invention concerns acetanilide derivatives of the formula I

$$R^4CON(R^5)\text{-}Ar^1\text{-}A^1\text{-}X^1\text{-}Ar^2\text{-}\underset{\displaystyle R^3}{\overset{\displaystyle OR^1}{C}}\text{-}R^2 \qquad \text{I}$$

wherein $R^4$ is (1-4C)alkyl;
$R^5$ is hydrogen or (1-4C)alkyl;
$Ar^1$ is phenylene, pyridinediyl or pyrimidinediyl;
$A^1$ is a direct link to $X^1$ or $A^1$ is (1-4C)alkylene;
$X^1$ is oxy, thio, sulphinyl or sulphonyl;
$Ar^2$ is thiophenediyl, furandiyl, thiazolediyl, oxazolediyl, thiadiazolediyl or oxadiazolediyl;
$R^1$ and $R^2$ together form a group of the formula $-A^2-X^2-A^3-$ which together with the oxygen atom to which $A^2$ is attached and with the carbon atom to which $A^3$ is attached define a ring having 5 or 6 ring atoms, wherein each of $A^2$ and $A^3$ is (1-3C)alkylene and $X^2$ is oxy, thio, sulphinyl or sulphonyl and which ring may bear one, two or three (1-4C)alkyl substituents; and
$R^3$ is (1-4C)alkyl;
or pharmaceutically-acceptable salts thereof;
processes for their manufacture; pharmaceutical compositions containing them and their use as 5-lipoxygenase inhibitors.

EP 0 610 032 A1

This invention concerns acetanilide derivatives and more particularly acetanilide derivatives which are inhibitors of the enzyme 5-lipoxygenase (hereinafter referred to as 5-LO). The invention also concerns processes for the manufacture of said acetanilide derivatives and novel pharmaceutical compositions containing them. Also included in the invention is the use of said acetanilide derivatives in the treatment of various inflammatory and/or allergic diseases in which the direct or indirect products of 5-LO catalysed oxidation of arachidonic acid are involved, and the production of new medicaments for such use.

As stated above the acetanilide derivatives described hereinafter are inhibitors of 5-LO, which enzyme is known to be involved in catalysing the oxidation of arachidonic acid to give rise via a cascade process to the physiologically active leukotrienes such as leukotriene $B_4$ ($LTB_4$) and the peptido-lipid leukotrienes such as leukotriene $C_4$ ($LTC_4$) and leukotriene $D_4$ ($LTD_4$) and various metabolites.

The biosynthetic relationship and physiological properties of the leukotrienes are summarised by G.W. Taylor and S.R. Clarke in Trends in Pharmacological Sciences, 1986, 7, 100-103. The leukotrienes and their metabolites have been implicated in the production and development of various inflammatory and allergic diseases such as inflammation of the joints (especially rheumatoid arthritis, osteoarthritis and gout), inflammation of the gastrointestinal tract (especially inflammatory bowel disease, ulcerative colitis and gastritis), skin disease (especially psoriasis, eczema and dermatitis) and respiratory disease (especially asthma, bronchitis and allergic rhinitis), and in the production and development of various cardiovascular and cerebrovascular disorders such as myocardial infarction, angina and peripheral vascular disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

It is disclosed in European Patent Application Nos. 0375457 A2 and 0385679 A2 that certain heterocyclic derivatives possess inhibitory properties against 5-LO. Furthermore European Patent Applications Nos. 0409412, 0409413 and 0462812 are also concerned with heterocyclic derivatives which possess inhibitory properties against 5-LO. We have now discovered that certain acetanilide derivatives which possess some structural features which are similar to those of the compounds disclosed in the above-mentioned applications but which possess other structural features, in particular acetanilide groups, which were not envisaged in those earlier applications are effective inhibitors of the enzyme 5-LO and thus of leukotriene biosyntheses. Thus such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided an acetanilide derivative of the formula I

$$R^4CON(R^5)\text{-}Ar^1\text{-}A^1\text{-}X^1\text{-}Ar^2\text{-}\underset{R^3}{\underset{|}{\overset{OR^1}{\overset{|}{C}}}}\text{-}R^2 \qquad I$$

wherein $R^4$ is (1-4C)alkyl;
$R^5$ is hydrogen or (1-4C)alkyl;
$Ar^1$ is phenylene, pyridinediyl or pyrimidinediyl which may optionally bear one or two substituents selected from halogeno, trifluoromethyl, hydroxy, (1-4C)alkyl and (1-4C)alkoxy;
$A^1$ is a direct link to $X^1$ or $A^1$ is (1-4C)alkylene;
$X^1$ is oxy, thio, sulphinyl or sulphonyl;
$Ar^2$ is thiophenediyl, furandiyl, thiazolediyl, oxazolediyl, thiadiazolediyl or oxadiazolediyl which may optionally bear one or two substituents selected from halogeno, trifluoromethyl, (1-4C)alkyl and (1-4C)alkoxy;
$R^1$ and $R^2$ together form a group of the formula $-A^2-X^2-A^3-$ which together with the oxygen atom to which $A^2$ is attached and with the carbon atom to which $A^3$ is attached define a ring having 5 or 6 ring atoms, wherein $A^2$ and $A^3$, which may be the same or different, each is (1-3C)alkylene and $X^2$ is oxy, thio, sulphinyl or sulphonyl and which ring may bear one, two or three (1-4C)alkyl substituents; and $R^3$ is (1-4C)alkyl;
or a pharmaceutically-acceptable salt thereof.

In this specification the generic term "alkyl" includes both straight-chain and branched-chain alkyl groups. However references to individual alkyl groups such as "propyl" are specific for the straight-chain version only

and references to individual branched-chain alkyl groups such as "isopropyl" are specific for the branched-chain version only. An analogous convention applies to other generic terms.

It is to be understood that, insofar as certain of the compounds of formula I defined above may exist in optically active or racemic forms by virtue of one or more asymmetric carbon atoms, the invention includes in its definition any such optically active or racemic form which possesses the property of inhibiting 5-LO. The synthesis of optically active forms may be carried out by standard techniques of organic chemistry well known in the art, for example by synthesis from optically active starting materials or by resolution of a racemic form.

Suitable values for the generic terms referred to above include those set out below.

A suitable value for $R^4$ or $R^5$ when it is (1-4C)alkyl is, for example, methyl, ethyl, propyl, isopropyl or butyl.

Suitable values for substituents on $Ar^1$ or $Ar^2$ include, for example:-

| | |
|---|---|
| for halogeno: | fluoro, chloro and bromo; |
| for (1-4C)alkyl: | methyl, ethyl, propyl and isopropyl; |
| for (1-4C)alkoxy: | methoxy, ethoxy, propoxy and isopropoxy. |

A suitable value for $Ar^1$ when it is phenylene is, for example, 1,3- or 1,4-phenylene.

A suitable value for $Ar^1$ when it is pyridinediyl or pyrimidinediyl is, for example, 2,4-, 2,5- or 3,5-pyridinediyl, or 2,5-or 4,6-pyrimidinediyl.

A suitable value for $A^1$ when it is (1-4C)alkylene is, for example, methylene, ethylene or trimethylene.

A suitable value for $Ar^2$ when it is thiophenediyl, furandiyl, thiazolediyl, oxazolediyl, thiadiazolediyl or oxadiazolediyl is, for example, 2,4- or 2,5-thiophenediyl, 2,4- or 2,5-furandiyl, 2,4- or 2,5-thiazolediyl, 2,4- or 2,5-oxazolediyl, 2,5-thiadiazolediyl or 2,5-oxadiazolediyl.

When $R^1$ and $R^2$ together form a group of the formula $-A^2-X^2-A^3-$ which together with the oxygen atom to which $A^2$ is attached and with the carbon atom to which $A^3$ is attached define a ring having 5 or 6 ring atoms then a suitable value for $A^2$ and $A^3$, which may be the same or different, when each is (1-3C)alkylene is, for example, methylene, ethylene or trimethylene. Suitable values for the (1-4C)alkyl substituents which may be present on said 5- or 6-membered ring include, for example, methyl, ethyl, propyl, isopropyl and butyl.

A suitable value for $R^3$ when it is (1-4C)alkyl is, for example, methyl, ethyl, propyl or butyl.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular compounds of the invention include, for example, acetanilide derivatives of the formula I, or pharmaceutically-acceptable salts thereof, wherein:-

(a) $Ar^1$ is phenylene which may optionally bear one or two substituents selected from halogeno, hydroxy, (1-4C)alkyl and (1-4C)alkoxy; and $R^4$, $R^5$, $A^1$, $X^1$, $Ar^2$, $R^1$, $R^2$ and $R^3$ have any of the meanings defined hereinbefore or in this section defining particular compounds;

(b) $Ar^1$ is 1,4-phenylene which may optionally bear a substituent selected from halogeno, hydroxy, (1-4C)alkyl and (1-4C)alkoxy; and $R^4$, $R^5$, $A^1$, $X^1$, $Ar^2$, $R^1$, $R^2$ and $R^3$ have any of the meanings defined hereinbefore or in this section defining particular compounds;

(c) $Ar^1$ is 2,5-pyridinediyl; and $R^4$, $R^5$, $A^1$, $X^1$, $Ar^2$, $R^1$, $R^2$ and $R^3$ have any of the meanings defined hereinbefore or in this section defining particular compounds;

(d) $A^1$ is a direct link to $X^1$; and $R^4$, $R^5$, $Ar^1$, $X^1$, $Ar^2$, $R^1$, $R^2$ and $R^3$ have any of the meanings defined hereinbefore or in this section defining particular compounds;

(e) $A^1$ is (1-4C)alkylene and $X^1$ is oxy; and $R^4$, $R^5$, $Ar^1$, $Ar^2$, $R^1$, $R^2$ and $R^3$ have any of the meanings defined hereinbefore or in this section defining particular compounds;

(f) $A^1$ is a direct link to $X^1$ and $X^1$ is thio; and $R^4$, $R^5$, $Ar^1$, $Ar^2$, $R^1$, $R^2$ and $R^3$ have any of the meanings defined hereinbefore or in this section defining particular compounds;

(g) $Ar^2$ is thiophenediyl; and $R^4$, $R^5$, $Ar^1$, $A^1$, $X^1$, $R^1$, $R^2$ and $R^3$ have any of the meanings defined hereinbefore or in this section defining particular compounds;

(h) $Ar^2$ is thiazolediyl; and $R^4$, $R^5$, $Ar^1$, $A^1$, $X^1$, $R^1$, $R^2$ and $R^3$ have any of the meanings defined hereinbefore or in this section defining particular compounds; or

(i) $R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein each of $A^2$ and $A^3$ is methylene and $X^2$ is oxy, and which ring may optionally bear one or two substituents selected from methyl and ethyl, and $R^3$ is methyl or ethyl; and $R^4$, $R^5$, $Ar^1$, $A^1$, $X^1$ and $Ar^2$ have any of the

meanings defined hereinbefore or in this section defining particular compounds.

A preferred compound of the invention comprises an acetanilide derivative of the formula I wherein $R^4$ is hydrogen, methyl, ethyl, propyl or isopropyl;
$R^5$ is hydrogen, methyl, ethyl, propyl or isopropyl;
$Ar^1$ is 1,4-phenylene which may optionally bear one substituent selected from fluoro, chloro, hydroxy, methyl and methoxy;
$A^1$ is a direct link to $X^1$ and $X^1$ is thio or sulphonyl, or $A^1$ is methylene and $X^1$ is oxy;
$Ar^2$ is 2,4- or 2,5-thiophenediyl or 2,4- or 2,5-thiazolediyl;
$R^1$ and $R^2$ together form a group of the formula $-A^2-X^2-A^3-$ which together with the oxygen atom to which $A^2$ is attached and the carbon atom to which $A^3$ is attached define a ring having 5 ring atoms wherein each of $A^2$ and $A^3$ is methylene and $X^2$ is oxy, and which ring may optionally bear one or two substituents selected from methyl and ethyl, and $R^3$ is methyl or ethyl;
or a pharmaceutically-acceptable salt thereof.

A further preferred compound of the invention comprises an acetanilide derivative of the formula I wherein $R^4$ is methyl or ethyl;
$R^5$ is hydrogen, methyl or ethyl;
$Ar^1$ is 1,4-phenylene;
$A^1$ is a direct link to $X^1$ and $X^1$ is thio;
$Ar^2$ is 2,4-thiophenediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2-$, $A^3$ is methylene and $X^2$ is oxy, and $R^3$ is methyl;
or a pharmaceutically-acceptable salt thereof.

A further preferred compound of the invention comprises an acetanilide derivative of the formula I wherein $R^4$ is methyl or ethyl;
$R^5$ is hydrogen, methyl or ethyl;
$Ar^1$ is 1,4-phenylene;
$A^1$ is a direct link to $X^1$ and $X^1$ is thio;
$Ar^2$ is 2,4-thiazolediyl (with the $X^1$ group in the 2-position) or 2,5-thiazolediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2$ , $A^3$ is methylene and $X^2$ is oxy, and $R^3$ is methyl;
or a pharmaceutically-acceptable salt thereof.

A further preferred compound of the invention comprises an acetanilide derivative of the formula I wherein $R^4$ is methyl;
$R^5$ is methyl;
$Ar^1$ is 1,4-phenylene or 2,5-pyridinediyl (with the $-A^1-X^1-$ group in the 2-position);
$A^1$ is a direct link to $X^1$ and $X^1$ is thio or sulphonyl;
$Ar^2$ is 2,4-thiophenediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2-$, $A^3$ is methylene and $X^2$ is oxy; and $R^3$ is methyl;
or a pharmaceutically-acceptable salt thereof.

A specific especially preferred compound of the invention is, for example, the following acetanilide derivative of the formula I, or a pharmaceutically-acceptable salt thereof:-
N-methyl-4'-{4-[(4S)-2,2,4-trimethyl-1,3-dioxolan-4-yl]thien-2-ylthio}-acetanilide or
N-methyl-4'-{5-[(4R)-2,2,4-trimethyl-1,3-dioxolan-4-yl]thiazol-2-ylthio}acetanilide.

A compound of the invention comprising an acetanilide derivative of the formula I, or a pharmaceutically-acceptable salt thereof, may be prepared by any process known to be applicable to the preparation of structurally-related compounds. Such procedures are provided as a further feature of the invention and are illustrated by the following representative examples in which, unless otherwise stated, $R^4$, $R^5$, $Ar^1$, $A^1$, $X^1$, $Ar^2$, $R^1$, $R^2$ and $R^3$ have any of the meanings defined hereinbefore, provided that when there is a hydroxy group in $Ar^1$ then any such group may optionally be protected by a conventional protecting group which may be removed when so desired by conventional means.

(a) The coupling, conveniently in the presence of a suitable base, of a compound of the formula II

$$R^4CON(R^5)-Ar^1-A^1-Z \qquad II$$

wherein Z is a displaceable group with a compound of the formula III

$$\begin{array}{c} OR^1 \\ | \\ HX^1\text{-}Ar^2\text{-}C\text{-}R^2 \\ | \\ R^3 \end{array} \qquad III$$

A suitable base for the reaction is, for example, an alkali or alkaline earth metal carbonate, (1-4C)alkoxide, (1-4C)alkanoate, hydroxide or hydride, for example sodium carbonate, potassium carbonate, barium carbonate, sodium ethoxide, potassium butoxide, sodium acetate, sodium hydroxide, potassium hydroxide, sodium hydride or potassium hydride. Alternatively a suitable base for the reaction is, for example, an organic amine base such as pyridine, 2,6-lutidine, collidine, 4-dimethylaminopyridine, triethylamine, N-methylmorpholine or diazabicyclo[5.4.0]undec-7-ene.

A suitable displaceable group Z is, for example, a halogeno or sulphonyloxy group, for example a chloro, bromo, iodo, methanesulphonyloxy or toluene-4-sulphonyloxy group.

The reaction is conveniently performed in a suitable inert solvent or diluent, for example, one or more of water, a (1-4C)alcohol such as methanol, ethanol and propanol, pyridine, 1,2-dimethoxyethane, tetrahydrofuran, 1,4-dioxan or a dipolar aprotic solvent such as N,N-dimethylformamide and dimethylsulphoxide. The reaction is conveniently performed at a temperature in the range, for example, 10 to 150°C, conveniently at or near 130°C.

The starting materials of the formulae II and III may be obtained by standard procedures of organic chemistry. The preparation of such starting materials is described within the accompanying non-limiting Examples. Alternatively necessary starting materials are obtainable by analogous procedures to those illustrated which are within the ordinary skill of an organic chemist. The disclosures of European Patent Applications Nos. 0375457, 0385679, 0409412, 0409413 and 0462812 are particularly relevant to the preparation of suitable starting materials.

(b) The cyclisation, conveniently in the presence of a suitable acid, of a compound of the formula IV

$$\begin{array}{c} \qquad\qquad\qquad\qquad\qquad\quad OH \\ \qquad\qquad\qquad\qquad\qquad\quad | \\ R^4CON(R^5)\text{-}Ar^1\text{-}A^1\text{-}X^1\text{-}Ar^2\text{-}C\text{-}A^3\text{-}X^2\text{-}H \\ \qquad\qquad\qquad\qquad\qquad\quad | \\ \qquad\qquad\qquad\qquad\qquad\quad R^3 \end{array} \qquad IV$$

with an appropriate aldehyde or ketone, or with the corresponding hemiacetal or acetal derivative thereof.

A suitable acid for the cyclisation reaction is, for example, an inorganic acid such as hydrochloric, sulphuric or phosphoric acid, or, for example, an organic acid such as 4-toluenesulphonic acid or trifluoroacetic acid. The cyclisation reaction is conveniently performed in a suitable inert solvent or diluent, for example 1,2-dimethoxyethane or tetrahydrofuran. Preferably the reaction is performed using the appropriate aldehyde or ketone as both a reactant and diluent. The cyclisation is effected at a temperature in the range, for example, 20 to 150°C, conveniently at or near the boiling point of the diluent or solvent.

The tertiary alcohol starting material of the formula IV may be obtained by standard procedures of organic chemistry. The preparation of examples of such starting materials is described within the accompanying non-limiting Examples which are provided for the purpose of illustration only.

(c) The coupling of a compound of the formula V

$$R^4CON(R^5)\text{-}Ar^1\text{-}A^1\text{-}X^1\text{-}Z \qquad V$$

wherein Z is a displaceable group as defined hereinbefore, or alternatively, when $X^1$ is a thio group, Z may be a group of the formula

$$R^4CON(R^5)\text{-}Ar^1\text{-}A^1\text{-}X^1\text{-}$$

with an organometallic reagent of the formula VI

$$\begin{array}{c} OR^1 \\ | \\ M\text{-}Ar^2\text{-}C\text{-}R^2 \\ | \\ R^3 \end{array} \qquad VI$$

wherein M is an alkali metal or alkaline earth metal such as lithium or calcium or M represents the magnesium halide portion of a conventional Grignard reagent.

The coupling reaction is conveniently performed in a suitable inert solvent or diluent as defined hereinbefore and at a temperature in the range, for example, -80 to +50°C, conveniently in the range -80°C to ambient temperature.

The preparation of the starting materials of the formulae V and VI may be obtained by standard procedures of organic chemistry.

(d) For the production of those compounds of the formula I wherein $X^1$ is a sulphinyl or sulphonyl group, or wherein $R^1$ and $R^2$ together form a group of the formula $-A^2-X^2-A^3-$ and $X^2$ is a sulphinyl or sulphonyl group, the oxidation of a compound of the formula I wherein $X^1$ is a thio group or wherein $R^1$ and $R^2$ together form a group of the formula $-A^2-X^2-A^3-$ and $X^2$ is a thio group.

A suitable oxidising group is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or tert-butyl methyl ether and at a temperature, for example, at or near ambient temperature, that is in the range 15 to 35°C. When a compound carrying a sulphinyl group is required a milder oxidising agent may also be used, for example sodium or potassium metaperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.

Conventional protecting groups for a hydroxy group which may be present in $Ar^1$ are set out hereinafter.

A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.

When a pharmaceutically-acceptable salt of a compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, or by resolution of a racemic form of said compound using a conventional procedure.

As stated previously, the compounds of the formula I are inhibitors of the enzyme 5-LO. The effects of this inhibition may be demonstrated using one or more of the standard procedures set out below:-

a) An in vitro assay system involving incubating a test compound with heparinised human blood, prior to challenge with the calcium ionophore A23187 and then indirectly measuring the inhibitory effects on 5-LO by assaying the amount of $LTB_4$ using specific radioimmunoassays described by Carey and Forder (Prostaglandins, Leukotrienes Med., 1986, 22, 57; Prostaglandins, 1984, 28, 666; Brit. J. Pharmacol., 1985, 84, 34P) which involves the use of a protein-$LTB_4$ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane $B_2(TxB_2)$ described by Carey and Forder (see above). This test provides an indication of the effects of a test compound against 5-LO and also cyclooxygenase in the presence

of blood cells and proteins. It permits the selectivity of the inhibitory effect on 5-LO or cyclooxygenase to be assessed.

b) An ex vivo assay system, which is a variation of test a) above, involving administration to a group of rats of a test compound (usually orally as the suspension produced when a solution of the test compound in dimethylsulphoxide is added to carboxymethylcellulose), blood collection, heparinisation, challenge with A23187 and radioimmunoassay of $LTB_4$ and $TxB_2$. This test provides an indication of the bioavailability of a test compound as an inhibitor of 5-LO or cyclooxygenase.

c) An in vivo system involving measuring the effects of a test compound administered orally against the liberation of $LTB_4$ induced by zymosan within an air pouch generated within the subcutaneous tissue of the back of male rats. The rats are anaesthetised and air pouches are formed by the injection of sterile air (20ml). A further injection of air (10ml) is similarly given after 3 days. At 6 days after the initial air injection the test compound is administered (usually orally as the suspension produced when a solution of the test compound in dimethylsulphoxide is added to hydroxypropylmethylcellulose), followed by the intrapouch injection of zymosan (1ml of a 1% suspension in physiological saline). After 3 hours the rats are killed, the air pouches are lavaged with physiological saline, and the specific radioimmunoassay described above is used to assay $LTB_4$ in the washings. This test provides an indication of inhibitory effects against 5-LO in an inflammatory milieu.

Although the pharmacological properties of the compounds of the formula I vary with structural changes as expected, in general compounds of the formula I possess 5-LO inhibitory effects at the following concentrations or doses in one or more of the above tests a)-c):-

Test a): $IC_{50}$ ($LTB_4$) in the range, for example, 0.01-40μM $IC_{50}$ ($TxB_2$) in the range, for example, 40-200μM;
Test b): oral $ED_{50}$($LTB_4$) in the range, for example, 0.1-100mg/kg;
Test c): oral $ED_{50}$($LTB_4$) in the range, for example, 0.1-50mg/kg.

No overt toxicity or other untoward effects are present in tests b) and/or c) when compounds of the formula I are administered at several multiples of their minimum inhibitory dose or concentration.

Thus, by way of example, the compound N-methyl-4'-{4-[(4S)-2,2,4-trimethyl-1,3-dioxolan-4-yl]thien-2-ylthio}acetanilide has an $IC_{50}$ of <0.04μM against $LTB_4$ in test a) and an oral $ED_{50}$ of approximately 1.5 mg/kg versus $LTB_4$ in test c); and the compound N-methyl-4'-{5-[(4R)-2,2,4-trimethyl-1,3-dioxolan-4-yl]thiazol-2-ylthio}acetanilide has an $IC_{50}$ of 0.07μM against $LTB_4$ in test a). In general those compounds of the formula I which are particularly preferred have an $IC_{50}$ of <1μM against $LTB_4$ in test a) and an oral $ED_{50}$ of <10 mg/kg against $LTB_4$ in tests b) and/or c).

These compounds are examples of compounds of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises an acetanilide derivative of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder such as a dry powder, a microcrystalline form or a liquid aerosol; for sub-lingual or buccal use, for example a tablet or capsule; or for parenteral use (including intravenous, subcutaneous, intramuscular, intravascular or infusion), for example a -sterile aqueous or oily solution or suspension.

In general the above compositions may be prepared in a conventional manner using conventional excipients.

The amount of active ingredient (that is an acetanilide derivative of the formula I, or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided an acetanilide derivative of the formula I, or a pharmaceutically-acceptable salt thereof, for use in a method of treatment of the human or animal body by therapy.

The invention also includes a method of treating a disease or medical condition mediated alone or in part by one or more leukotrienes which comprises administering to a warm-blooded animal requiring such treatment an effective amount of an active ingredient as defined above. The invention also provides the use of such an active ingredient in the production of a new medicament for use in a leukotriene mediated disease or medical condition.

The size of the dose for therapeutic or prophylactic purposes of a compound of the formula I will naturally vary according to the nature and severity of the conditions, the age and sex of the animal or patient and the route of administration, according to well known principles of medicine. As mentioned above, compounds of the formula I are useful in treating those allergic and inflammatory conditions which are due alone or in part to the effects of the metabolites of arachidonic acid arising by the linear (5-LO catalysed) pathway and in particular the leukotrienes, the production of which is mediated by 5-LO. As previously mentioned, such conditions include, for example, -asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5 mg to 75 mg per kg body weight is received, given if required in divided doses. In general lower doses will be administered when a parenteral route is employed. Thus, for example, for intravenous administration, a dose in the range, for example, 0.5 mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises an acetanilide derivative of the formula I, or a pharmaceutically-acceptable salt thereof as defined hereinbefore, in conjunction or admixture with a cyclooxygenase inhibitory non-steroidal anti-inflammatory agent (such as those mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The invention will now be illustrated in the following non-limiting Examples in which, unless otherwise stated:-

(i) evaporations were carried out by rotary evaporation in vacuo and work-up procedures were carried out after removal of residual solids by filtration;

(ii) operations were carried out at ambient temperature, that is in the range 18-25°C and under an atmosphere of an inert gas such as argon;

(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Merck, Darmstadt, Germany;

(iv) yields are given for illustration only and are not necessarily the maximum attainable;

(v) the structures of the end-products of the formula I were confirmed by NMR and mass spectral techniques; unless otherwise stated, $CDCl_3$ solutions of the end-products of the formula I were used for the determination of the NMR spectral data, chemical shift values were measured on the delta scale and the following abbreviations are used: s, singlet; d, doublet; t, triplet; q, quartet; m, multiplet;

(vi) intermediates were not generally fully characterised and purity was assessed by thin layer chromatographic, infra-red (IR) or NMR analysis;

(vii) melting points are uncorrected and were determined using a Mettler SP62 automatic melting point apparatus or an oil-bath apparatus; melting points for the end-products of the formula I were determined after recrystallisation from a conventional organic solvent such as ethanol, methanol, acetone, ether or hexane, alone or in admixture;
(viii) the following abbreviations have been used:-
THF tetrahydrofuran;
DMF N,N-dimethylformamide.

**Example 1**

A mixture of N-methyl-4'-iodoacetanilide (0.52 g), 2-mercapto-4-[(4S)-2,2,4-trimethyl-1,3-dioxolan-4-yl]thiophene (0.48 g), potassium carbonate (0.4 g), cuprous chloride (0.055 g) and DMF (5 ml) was stirred and heated at 130°C for 2 hours. The mixture was cooled to ambient temperature and partitioned between ethyl acetate and water. The organic phase was washed with water and with brine, dried ($MgS0_4$) and evaporated. The residue was purified by column chromatography using a 5:1 mixture of methylene chloride and ethyl acetate as eluent. There was thus obtained N-methyl-4'-{4-[(4S)-2,2,4-trimethyl-1,3-dioxolan-4-yl]thien-2-ylthio}acetanilide (0.68 g, 90%) as an oil;
NMR Spectrum: ($CD_3SOCD_3$) 1.33 (s, 3H), 1.4 (s, 3H), 1.53 (s, 3H), 1.7-1.9 (s, 3H); 3.3 (s, 3H), 3.95-4.06 (q, 2H), 7.2-7.35 (m, 4H), 7.4 (d, 1H), 7.65 (d, 1H);
Elemental Analysis: Found C, 60.1; H, 6.1; N, 3.4;
$C_{19}H_{23}NO_3S_2$ requires C, 60.4; H, 6.1; N, 3.7%.

The N-methyl-4'-iodoacetanilide used as a starting material was obtained as follows:-

Acetyl chloride (1.84 g) was added dropwise to a stirred mixture of 4-iodoaniline (5 g), triethylamine (3.5 ml) and methylene chloride (50 ml) which had been cooled to 10°C in an ice-bath. The mixture was stirred at 10°C for 3 hours. The mixture was partitioned between ethyl acetate and water. The organic phase was washed with water and with brine, dried ($MgSO_4$) and evaporated. There was thus obtained 4'-iodoacetanilide (5.52 g), m.p. 179-181°C.

Sodium hydride (60% dispersion in mineral oil, 0.92 g) was added portionwise to a stirred solution of 4'-iodoacetanilide (5.5 g) in DMF (35 ml) which had been cooled to 10°C in an ice-bath. The mixture was stirred and allowed to warm to ambient temperature during 2 hours. Methyl iodide (1.6 ml) was added and the mixture was stirred at ambient temperature for 66 hours. The mixture was poured onto a mixture of dilute aqueous hydrochloric acid and ice. The precipitate was isolated, washed in turn with water and petroleum ether and dried. There was thus obtained N-methyl-4'-iodoacetanilide (5.1 g), m.p. 140-142°C;
NMR Spectrum: ($CD_3SOCD_3$) 1.8 (s, 3H), 3.16 (s, 3H), 7.15 (d, 2H), 7.85 (d, 2H).

The 2-mercapto-4-[(4S)-2,2,4-trimethyl-1,3-dioxolan-4-yl]-thiophene used as a starting material was obtained as follows:-

n-Butyl-lithium (1.55M in hexane, 37 ml) was added to a stirred mixture of methyltriphenylphosphonium bromide (21.4 g) and THF (100 ml) which had been cooled to 10°C. The mixture was stirred at ambient temperature for 90 minutes. The mixture was recooled in an ice-bath and a solution of 4-acetyl-2-bromothiophene (Synth. Comm., 1981, 29-34; 11 g) in THF (60 ml) was added. The mixture was stirred at 0°C for 1 hour and at ambient temperature for 2 hours. The mixture was partitioned between diethyl ether and a saturated aqueous ammonium chloride solution. The organic phase was washed with water and with brine, dried ($MgSO_4$) and evaporated. The residue was purified by column chromatography using hexane as eluent. There was thus obtained 2-bromo-4-isopropenylthiophene (9 g, 85%).

n-Butyl-lithium (1.6M in THF, 6.1 ml) was added to a stirred solution of a portion (1.85 g) of the product so obtained in diethyl ether (20 ml) which had been cooled to -80°C. The mixture was stirred at -80°C for 1 hour. A solution of dimethyl disulphide (0.942 g) in diethyl ether (5 ml) was added. The mixture was stirred and allowed to warm to -40°C during 2 hours. The mixture was poured onto a mixture of a saturated solution of ammonium chloride and ice and extracted with diethyl ether. The organic phase was washed with water and with brine, dried ($MgSO_4$) and evaporated. The residue was purified by column chromatography using hexane as eluent. There was thus obtained 4-isopropenyl-2-methylthiothiophene (1.35 g, 87%);
NMR Spectrum: 2.08 (s, 3H), 2.5 (s, 3H), 5.0 (m, 1H), 5.3 (m, 1H), 7.13 (d, 1H), 7.25 (d, 1H).

After repetition of the previous reaction, the compound so obtained (3.1 g) was added to a vigorously stirred mixture of an osmium dihydroquinine complex known as AD-mix-a (25.2 g; J. Org. Chem., 1992, 57, 2768-2771), tert-butanol (90 ml) and water (90 ml) which had been cooled to 0°C. The mixture was stirred at 0°C for 6 hours and stored at -5°C for 16 hours. Sodium sulphite (10 g) was added and the mixture was stirred and allowed to warm to ambient temperature. The mixture was partitioned between ethyl acetate and water. The organic phase was dried ($MgSO_4$) and evaporated. The residue was purified by column chromatography

using a 5:3 mixture of hexane and ethyl acetate as eluent. There was thus obtained 4-[(2S)-1,2-dihydroxyprop-2-yl]-2-methylthiothiophene (3.41 g, 92%), m.p. 71-73°C, [alpha]$^{25}$ = + 14.8° (conc. = 0.5 g per 100 ml of methylene chloride).

A mixture of the product so obtained, acetone dimethyl acetal (2.6 g), 4-toluenesulphonic acid (0.04 g) and acetone (50 ml) was stirred and heated to reflux for 1 hour. The mixture was cooled to ambient temperature and neutralised by the addition of dilute aqueous potassium carbonate solution. The mixture was partitioned between ethyl acetate and water. The organic phase was washed with water, dried ($MgSO_4$) and evaporated. The residue was purified by column chromatography using a 19:1 mixture of hexane and ethyl acetate as eluent. There was thus obtained 2-methylthio-4-[(4S)-2,2,4-trimethyl-1,3-dioxolan-4-yl]thiophene in 87% yield as an oil (3.57 g);
NMR Spectrum: ($CD_3SOCD_3$) 1.3 (s, 3H), 1.4 (s, 3H), 1.5 (s, 3H), 2.5 (s, 3H), 3.9-4.0 (q, 2H), 7.1 (d, 1H), 7.3 (d, 1H).

A mixture of a portion (0.5 g) of the product so obtained, sodium methanethiolate (0.5 g) and DMF (5 ml) was stirred and heated to 130°C for 35 minutes. The mixture was cooled to ambient temperature and partitioned between ethyl acetate and water. The mixture was acidified by the addition of 1M aqueous citric acid solution. The organic layer was washed with water and with brine, dried ($MgSO_4$) and evaporated. There was thus obtained 2-mercapto-4-[(4S)-2,2,4-trimethyl-1,3-dioxolan-4-yl]thiophene (0.48 g).

## Example 2

Using an analogous procedure to that described in Example 1, N-methyl-4'-iodoacetanilide was reacted with 2-mercapto-5-[(4R)-2,2,4-trimethyl-1,3-dioxolan-4-yl]thiazole to give N-methyl-4'-{5-[(4R)-2,2,4-trimethyl-1,3-dioxolan-4-yl]thiazol-2-ylthio}acetanilide in 72% yield, m.p. 65-70°C;
NMR Spectrum: ($CD_3SOCD_3$) 1.3 (s, 3H), 1.35 (s, 3H), 1.6 (s, 3H), 1.85-1.90 (s, 3H), 3.3 (s, 3H), 4.0 (m, 2H), 7.45 (d, 2H), 7.65 (d, 2H), 7.7 (s, 1H).

The 2-mercapto-5-[(4R)-2,2,4-trimethyl-l,3-dioxolan-4-yl]-thiazole used as a starting material was obtained as follows:-

A mixture of 2-bromothiazole (32.8 g), potassium iodide (2 g), sodium methanethiolate (14.7 g) and methanol (200 ml) was stirred and heated to reflux for 8 hours. The bulk of the solvent was evaporated and the residue was partitioned between diethyl ether and water. The organic phase was washed with water and with brine, dried ($MgSO_4$) and evaporated. The residue was distilled to give 2-methylthiothiazole (18.2 g) as an oil (b.p. 58-60°C at 10 mm Hg).

n-Butyl-lithium (1.55M in hexane, 30 ml) was added to a stirred solution of 2-methylthiothiazole (5.4 g) in diethyl ether which had been cooled to -10°C. The mixture was stirred at -10°C for 20 minutes. A solution of acetone (9.2 ml) in diethyl ether (10 ml) was added. The mixture was stirred at -5°C for 4 hours. The mixture was partitioned between diethyl ether and a saturated aqueous ammonium chloride solution. The organic layer was washed with brine, dried ($MgSO_4$) and evaporated. The residue was purified by column chromatography using a 3:2 mixture of hexane and ethyl acetate as eluent. There was thus obtained 5-(2-hydroxyprop-2-yl)-2-methyl- thiothiazole (6.2 g, 80%) as an oil.

A mixture of the product so obtained, triethyl orthoformate (5.8 ml), 4-toluenesulphonic acid (0.065 g) and ethanol (40 ml) was stirred and heated to reflux for 1 hour. The bulk of the ethanol was evaporated and the residue was partitioned between diethyl ether and water. The organic phase was washed with brine, dried ($MgSO_4$) and evaporated. The residue was purified by column chromatography using a 10:1 mixture of toluene and ethyl acetate as eluent. There was thus obtained 5-(2-ethoxyprop-2-yl)-2-methylthiothiazole (5.2 g, 65%) as an oil.

A mixture of a portion (4.37 g) of the material so obtained, boron trifluoride ether complex (6 ml) and methylene chloride (10 ml) was stirred at ambient temperature for 3 hours. The mixture was evaporated and the residue was partitioned between diethyl ether and a saturated aqueous potassium carbonate solution. The organic phase was washed with brine, dried ($MgSO_4$) and evaporated. The residue was purified by column chromatography using a 50:3 mixture of hexane and ethyl acetate as eluent. There was thus obtained 5-isopropenyl-2-methylthiothiazole (2.55 g, 74%) as an oil.
NMR Spectrum: 2.1 (s, 3H), 2.7 (s, 3H), 5.0-5.2 (m, 2H), 7.5 (s, 1H).

Using analogous procedures to those described in the third to fifth paragraphs of the portion of Example 1 which is concerned with the preparation of starting materials, the product so obtained was converted in turn into:-
5-[(2R)-1,2-dihydroxyprop-2-yl]-2-methylthiothiazole in 83% yield, m.p. 107-109°C;
2-methylthio-5-[(4R)-2,2,4-trimethyl-1,3-dioxolan-4-yl]thiazole in 88% yield as an oil,
NMR Spectrum: ($CD_3SOCD_3$) 1.3 (s, 3H), 1.4 (s, 3H), 1.6 (s, 3H), 2.65 (s, 3H), 4.0-4.1 (q, 2H), 7.55 (s, 1H);

and
2-mercapto-5-[(4R)-2,2,4-trimethyl-l,3-dioxolan-4-yl]thiazole in quantitative yield.

## Example 3

The procedure described in Example 1 was repeated except that racemic 2-mercapto-4-(2,2,4-trimethyl-1,3-dioxolan-4-yl)thiophene was used in place of 2-mercapto-4-[(2S)-2,2,4-trimethyl-1,3-dioxolan-4-yl]-thiophene. There was thus obtained N-methyl-4'-[4-(2,2,4-trimethyl-1,3-dioxolan-4-yl)thien-2-ylthio)acetanilide in 69% yield;
NMR Spectrum: ($CD_3SOCD_3$) 1.33 (s, 3H), 1.4 (s, 3H), 1.53 (s, 3H), 1.7-1.9 (s, 3H), 3.3 (s, 3H), 3.95-4.06 (q, 2H), 7.2-7.35 (m, 4H), 7.4 (d, 1H), 7.65 (d, 1H).

The racemic 2-mercapto-4-(2,2,4-trimethyl-1,3-dioxolan-4-yl)-thiophene used as a starting material was obtained as follows:-

n-Butyl-lithium (1.6M in THF, 28.5 ml) was added to a stirred mixture of 2,4-dibromothiophene (J. Org. Chem., 1988, 53, 417; 10 g) and diethyl ether (150 ml) which had been cooled to -70°C. After 1 hour, a solution of dimethyl disulphide (3 ml) in diethyl ether (10 ml) was added and the mixture was stirred and allowed to warm to -20°C during 1 hour. The mixture was poured into water. The organic phase was washed with brine, dried ($MgSO_4$) and evaporated. The residue was purified by column chromatography using hexane as eluent. There was thus obtained 4-bromo-2-methylthiothiophene (4.76 g, 90%).

n-Butyl-lithium (1.5M in hexane, 16.6 ml) was added to a stirred solution of 4-bromo-2-methylthiothiophene (5.2 g) in diethyl ether (150 ml) which had been cooled to -78°C. The mixture was stirred at -70°C for 1 hour. A solution of 1-tetrahydropyran-2-yloxypropan-2-one (4.3 g) in diethyl ether (5 ml) was added. The mixture was stirred at -70°C for 1 hour and then allowed to warm to -30°C. The mixture was poured onto a mixture of ice and a saturated aqueous solution of ammonium chloride. The mixture was extracted with diethyl ether. The organic layer was washed with brine, dried ($MgSO_4$) and evaporated. The residue was purified by column chromatograpny using a 10:3 mixture of hexane and ethyl acetate as eluent. There was thus obtained 4-(2-hydroxy-1-tetrahydropyran-2-yloxyprop-2-yl)-2-methylthiophene (5.35 g, 75%) as an oil.

A mixture of the product so obtained, 2N aqueous hydrochloric acid (3 ml) and methanol (20 ml) was stirred at ambient temperature for 1.5 hours. The bulk of the methanol was evaporated and the residue was partitioned between ethyl acetate and a dilute aqueous potassium carbonate solution. The organic phase was washed with brine, dried ($MgSO_4$) and evaporated. The residue was purified by column chromatography using a 2:1 mixture of hexane and ethyl acetate as eluent. There was thus obtained 4-(1,2-dihydroxyprop-2-yl)-2-methylthiothiophene (3.08 g, 82%), m.p. 40-42°C.

A mixture of a portion (1 g) of the compound so obtained, acetone dimethyl acetal (1 ml), 4-toluenesulphonic acid (0.01 g) and acetone (12 ml) was stirred and heated to reflux for 45 minutes. The mixture was cooled to ambient temperature and neutralised by the addition of a dilute aqueous potassium carbonate solution. The mixture was extracted with ethyl acetate. The organic phase was washed with brine, dried ($MgSO_4$) and evaporated. The residue was purified by column chromatography using a 20:1 mixture of hexane and ethyl acetate as eluent. There was thus obtained
4-(2,2,4-trimethyl-1,3-dioxolan-4-yl)-2-methylthiothiophene (0.97 g, 81%) as an oil.

A mixture of a portion (0.347 g) of the material so obtained, sodium methanethiolate (0.35 g) and DMF (4 ml) was stirred and heated to 130°C for 30 minutes. The mixture was cooled to ambient temperature and partitioned between diethyl ether and a dilute aqueous citric acid solution. The organic phase was washed with brine, dried ($MgSO_4$) and evaporated. There was thus obtained 2-mercapto-4-(2,2,4-trimethyl-1,3-dioxolan-4-yl)thiophene as an oil.

## Example 4

Sufficient sodium acetate was added to a solution of potassium peroxymonosulphate (0.3 g) in water (2 ml) in order to bring the acidity of the solution to pH5-6. The resultant solution was added dropwise to a stirred solution of N-methyl-4'-[4-(2,2,4-trimethyl-1,3-dioxolan-4-yl)thien-2-ylthio]acetanilide (0.13 g) in methanol (4 ml) which had been cooled to 0°C. The mixture was allowed to warm to ambient temperature and was stirred for 4 hours. The mixture was partitioned between ethyl acetate and water. The organic phase was washed with brine, dried ($MgSO_4$) and evaporated. The residue was purified by column chromatography using increasingly polar mixtures of toluene and ethyl acetate as eluent. There was thus obtained N-methyl-4'-[4-(2,2,4-trimethyl-1,3-dioxolan-4-yl)thien-2-ylsulphonyl]-acetanilide (0.114 g, 80%);
NMR Spectrum: ($CD_3SOCD_3$) 1.3 (s, 3H), 1.4 (s, 3H), 1.5 (s, 3H), 1.95 (s, 3H), 3.2 (s, 3H), 3.9-4.1 (m, 2H), 7.6 (m, 2H), 7.85 (d, 1H), 7.9 (d, 1H), 7.95-8.05 (m, 2H).

**Example 5**

Using an analogous procedure to that described in Example 1, 2-chloro-5-(N-methylacetamido)pyridine was reacted with 2-mercapto-4-(2,2,4-trimethyl-1,3-dioxolan-4-yl)thiophene to give 5-(N-methylacetamido)-2-[4-(2,2,4-trimethyl-1,3-dioxolan-4-yl)thien-2-ylthio]pyridine in 15% yield;
NMR Spectrum: ($CD_3SOCD_3$ + $CD_3CO_2D$) 1.38 (s, 3H), 1.45 (s, 3H), 1.6 (s, 3H), 1.85 (s, 3H), 3.15 (s, 3H), 4.0-4.1 (m, 2H), 7.0 (d, 1H), 7.4 (d, 1H), 7.7 (m, 2H), 8.4 (d, 1H).

The 2-chloro-5-(N-methylacetamido)pyridine used as a starting material was obtained as follows:-

A solution of acetyl chloride (3 ml) in methylene chloride (10 ml) was added dropwise to a stirred mixture of 5-amino-2-chloropyridine (5 g), triethylamine (6 ml) and methylene chloride (60 ml) which had been cooled to 0°C. The mixture was allowed to warm to ambient temperature and was stirred for 2 hours. The mixture was partitioned between dilute aqueous sodium hydroxide solution and methylene chloride. The organic phase was washed with water, dried ($MgSO_4$) and evaporated. There was thus obtained 5-acetamido-2-chloropyridine (6.2 g, 93%), m.p. 145-148°C.

Sodium hydride (60% dispersion in mineral oil, 0.92 g) was added portionwise to a stirred solution of 5-acetamido-2-chloropyridine (4 g) in DMF (40 ml) which was cooled to 5°C. The mixture was stirred at 5°C for 1 hour. The mixture was cooled to 0°C and methyl iodide (1.43 ml) was added. The mixture was allowed to warm to ambient temperature and was stirred for 3 hours. The mixture was partitioned between ethyl acetate and water. The organic phase was washed with water, dried ($MgSO_4$) and evaporated. The residue was purified by column chromatography using ethyl acetate as eluent. There was thus obtained 2-chloro-5-(N-methylacetamido)pyridine (1.8 g, 42%), m.p. 42-45°C.

**Example 6**

The following illustrate representative pharmaceutical dosage forms containing the compound of formula I, or a pharmaceutically-acceptable salt thereof (hereafter compound X), for therapeutic or prophylactic use in humans:

(a)

| Tablet I | mg/tablet |
|---|---|
| Compound X | 100 |
| Lactose Ph.Eur | 182.75 |
| Croscarmellose sodium | 12.0 |
| Maize starch paste (5% w/v paste) | 2.25 |
| Magnesium stearate | 3.0 |

(b)

| Tablet II | mg/tablet |
|---|---|
| Compound X | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

(c) Tablet III

| Tablet III | mg/tablet |
|---|---|
| Compound X | 1.0 |
| Lactose Ph.Eur | 93.25 |
| Croscarmellose sodium | 4.0 |
| Maize starch paste (5% w/v paste) | 0.75 |
| Magnesium stearate | 1.0 |

(d) Capsule

| Capsule | mg/capsule |
|---|---|
| Compound X | 10 |
| Lactose Ph.Eur | 488.5 |
| Magnesium stearate | 1.5 |

(e) Injection I

| Injection I | (50 mg/ml) |
|---|---|
| Compound X | 5.0% w/v |
| 1M Sodium hydroxide solution | 15.0% v/v |
| 0.1M Hydrochloric acid (to adjust pH to 7.6) | |
| Polyethylene glycol 400 | 4.5% w/v |
| Water for injection to 100% | |

(f) Injection II

| Injection II | (10 mg/ml) |
|---|---|
| Compound X | 1.0% w/v |
| Sodium phosphate BP | 3.6% w/v |
| 0.1M Sodium hydroxide solution | 15.0% v/v |
| Water for injection to 100% | |

(g) Injection III

| Injection III | (1mg/ml, buffered to pH6) |
|---|---|
| Compound X | 0.1% w/v |
| Sodium phosphate BP | 2.26% w/v |
| Citric acid | 0.38% w/v |
| Polyethylene glycol 400 | 3.5% w/v |
| Water for injection to 100% | |

(h)

| Aerosol I | mg/ml |
|---|---|
| Compound X | 10.0 |
| Sorbitan trioleate | 13.5 |
| Trichlorofluoromethane | 910.0 |
| Dichlorodifluoromethane | 490.0 |

(i)

| Aerosol II | mg/ml |
|---|---|
| Compound X | 0.2 |
| Sorbitan trioleate | 0.27 |
| Trichlorofluoromethane | 70.0 |
| Dichlorodifluoromethane | 280.0 |
| Dichlorotetrafluoroethane | 1094.0 |

(j)

| Aerosol III | mg/ml |
|---|---|
| Compound X | 2.5 |
| Sorbitan trioleate | 3.38 |
| Trichlorofluoromethane | 67.5 |
| Dichlorodifluoromethane | 1086.0 |
| Dichlorotetrafluoroethane | 191.6 |

(k)

| Aerosol IV | mg/ml |
|---|---|
| Compound X | 2.5 |
| Soya lecithin | 2.7 |
| Trichlorofluoromethane | 67.5 |
| Dichlorodifluoromethane | 1086.0 |
| Dichlorotetrafluoroethane | 191.6 |

Note

The above formulations may be obtained by conventional procedures well known in the pharmaceutical art. The tablets (a)-(c) may be enteric coated by conventional means, for example to provide a coating of cellulose acetate phthalate. The aerosol formulations (h)-(k) may be used in conjunction with standard, metered dose aerosol dispensers, and the suspending agents sorbitan trioleate and soya lecithin may be replaced by an alternative suspending agent such as sorbitan monooleate, sorbitan sesquioleate, polysorbate 80, polyglycerol oleate or oleic acid.

## Claims

1. An acetanilide derivative of the formula I

$$\begin{array}{rcl} & OR^1 & \\ & | & \\ R^4CON(R^5)-Ar^1-A^1-X^1-Ar^2- & C & -R^2 \\ & | & \\ & R^3 & \end{array} \qquad I$$

wherein $R^4$ is (1-4C)alkyl;
$R^5$ is hydrogen or (1-4C)alkyl;
$Ar^1$ is phenylene, pyridinediyl or pyrimidinediyl which may optionally bear one or two substituents selected from halogeno, trifluoromethyl, hydroxy, (1-4C)alkyl and (1-4C)alkoxy;
$A^1$ is a direct link to $X^1$ or $A^1$ is (1-4C)alkylene;
$X^1$ is oxy, thio, sulphinyl or sulphonyl;
$Ar^2$ is thiophenediyl, furandiyl, thiazolediyl, oxazolediyl, thiadiazolediyl or oxadiazolediyl which may optionally bear one or two substituents selected from halogeno, trifluoromethyl, (1-4C)alkyl and (1-4C)alkoxy;
$R^1$ and $R^2$ together form a group of the formula $-A^2-X^2-A^3-$ which together with the oxygen atom to which $A^2$ is attached and with the carbon atom to which $A^3$ is attached define a ring having 5 or 6 ring atoms, wherein $A^2$ and $A^3$, which may be the same or different, each is (1-3C)alkylene and $X^2$ is oxy, thio, sulphinyl or sulphonyl and which ring may bear one, two or three (1-4C)alkyl substituents; and
$R^3$ is (1-4C)alkyl;
or a pharmaceutically-acceptable salt thereof.

2. An acetanilide derivative of the formula I as claimed in claim 1
wherein $R^4$ is hydrogen, methyl, ethyl, propyl or isopropyl;
$R^5$ is hydrogen, methyl, ethyl, propyl or isopropyl;
$Ar^1$ is 1,4-phenylene which may optionally bear one substituent selected from fluoro, chloro, hydroxy, methyl and methoxy;
$A^1$ is a direct link to $X^1$ and $X^1$ is thio or sulphonyl, or $A^1$ is methylene and $X^1$ is oxy;
$Ar^2$ is 2,4- or 2,5-thiophenediyl or 2,4- or 2,5-thiazolediyl;
$R^1$ and $R^2$ together form a group of the formula $-A^2-X^2-A^3-$ which together with the oxygen atom to which $A^2$ is attached and the carbon atom to which $A^3$ is attached define a ring having 5 ring atoms wherein each

of $A^2$ and $A^3$ is methylene and $X^2$ is oxy, and which ring may optionally bear one or two substituents selected from methyl and ethyl, and $R^3$ is methyl or ethyl;
or a pharmaceutically-acceptable salt thereof.

**3.** An acetanilide derivative of the formula I as claimed in claim 1
wherein $R^4$ is methyl or ethyl;
$R^5$ is hydrogen, methyl or ethyl;
$Ar^1$ is 1,4-phenylene;
$A^1$ is a direct link to $X^1$ and $X^1$ is thio;
$Ar^2$is 2,4-thiophenediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2-$, $A^3$ is methylene and $X^2$ is oxy, and $R^3$ is methyl;
or a pharmaceutically-acceptable salt thereof.

**4.** An acetanilide derivative of the formula I as claimed in claim 1
wherein $R^4$ is methyl or ethyl;
$R^5$ is hydrogen, methyl or ethyl;
$Ar^1$ is 1,4-phenylene;
$A^1$ is a direct link to $X^1$ and $X^1$ is thio;
$Ar^2$ is 2,4-thiazolediyl (with the $X^1$ group in the 2-position) or 2,5-thiazolediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2-$, $A^3$ is methylene and $X^2$ is oxy, and $R^3$ is methyl;
or a pharmaceutically-acceptable salt thereof.

**5.** An acetanilide derivative of the formula I as claimed in claim 1
wherein $R^4$ is methyl;
$R^5$ is methyl;
$Ar^1$ is 1,4-phenylene or 2,5-pyridinediyl (with the $-A^1-X^1-$ group in the 2-position);
$A^1$ is a direct link to $X^1$ and $X^1$ is thio or sulphonyl;
$Ar^2$ is 2,4-thiophenediyl (with the $X^1$ group in the 2-position);
$R^1$ and $R^2$ together form a group of the formula $-A^2-X^2-A^3-$ which together with the oxygen atom to which $A^2$ is attached and with the carbon atom to which $A^3$ is attached define a ring having 5 ring atoms, wherein $A^2$ is $-C(Me)_2-$, $A^3$ is methylene and $X^2$ is oxy; and
$R^3$ is methyl;
or a pharmaceutically-acceptable salt thereof.

**6.** An acetanilide derivative of the formula I, or a pharmaceutically-acceptable salt thereof, as claimed in claim 1 selected from:-
N-methyl-4'-{4-[(4S)-2,2,4-trimethyl-1,3-dioxolan-4-yl]thien-2-ylthio}-acetanilide and
N-methyl-4'-{5-[(4R)-2,2,4-trimethyl-1,3-dioxolan-4-yl]thiazol-2- ylthio}acetanilide.

**7.** A process for the preparation of an acetanilide derivative of the formula I, or a pharmaceutically-acceptable salt thereof, as claimed in any one of claims 1 to 6 which comprises:-
(a) the coupling of a compound of the formula II

$$R^4CON(R^5)-Ar^1-A^1-Z \qquad II$$

wherein Z is a displaceable group with a compound of the formula III

$$HX^1-Ar^2-\underset{R^3}{\overset{OR^1}{C}}-R^2 \qquad III$$

(b) the cyclisation of a compound of the formula IV

$$R^4CON(R^5)-Ar^1-A^1-X^1-Ar^2-\underset{R^3}{\overset{OH}{C}}-A^3-X^2-H \qquad IV$$

with an appropriate aldehyde or ketone, or with the corresponding hemiacetal or acetal derivative thereof;

(c) the coupling of a compound of the formula V

$$R^4CON(R^5)-Ar^1-A^1-X^1-Z \qquad V$$

wherein Z is a displaceable group, or alternatively, when $X^1$ is a thio group, Z may be a group of the formula

$$R^4CON(R^5)-Ar^1-A^1-X^1-$$

with an organometallic reagent of the formula VI

$$M-Ar^2-\underset{R^3}{\overset{OR^1}{C}}-R^2 \qquad VI$$

wherein M is an alkali metal or alkaline earth metal or M represents the magnesium halide portion of a conventional Grignard reagent; or

(d) for the production of those compounds of the formula I wherein $X^1$ is a sulphinyl or sulphonyl group, or wherein $R^1$ and $R^2$ together form a group of the formula $-A^2-X^2-A^3-$ and $X^2$ is a sulphinyl or sulphonyl group, the oxidation of a compound of the formula I wherein $X^1$ is a thio group or wherein $R^1$ and $R^2$ together form a group of the formula $-A^2-X^2-A^3-$ and $X^2$ is a thio group;

and when a pharmaceutically-acceptable salt of a compound of the formula I is required, it may be obtained by reaction of said compound with a suitable acid or base using a conventional procedure; and when an optically active form of a compound of the formula I is required, it may be obtained by carrying out one of the aforesaid procedures using an optically active starting material, or by resolution of a racemic form of said compound using a conventional procedure.

8. A pharmaceutical composition which comprises an acetanilide derivative of the formula I, or a pharmaceutically-acceptable salt thereof, as claimed in any one of claims 1 to 6 in association with a pharmaceutically-acceptable diluent or carrier.

9. A method of treating a disease or medical condition mediated alone or in part by one or more leukotrienes which comprises administering to a warm-blooded animal requiring such treatment an effective amount of an acetanilide derivative of the formula I, or a pharmaceutically-acceptable salt thereof, as claimed in

any one of claims 1 to 6.

**10.** The use of an acetanilide derivative of the formula I, or a pharmaceutically-acceptable salt thereof, as claimed in any one of claims 1 to 6 in the production of a new medicament for use in a leukotriene mediated disease or medical condition.

European Patent Office

# PARTIAL EUROPEAN SEARCH REPORT

which under Rule 45 of the European Patent Convention shall be considered, for the purposes of subsequent proceedings, as the European search report

Application Number EP 94 30 0629

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,A | EP-A-0 462 812 (I.C.I.)<br>* the whole document *<br>----- | 1,7,8,10 | C07D409/04<br>C07D417/04<br>C07D409/14<br>A61K31/38 |

TECHNICAL FIELDS SEARCHED (Int.Cl.5)

C07D

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 27 April 1994 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 94 30 0629
-C-

Remark: Although claim 9
is directed to a method of
treatment of the human/animal
body (Art. 52(4) EPC) the search
has been carried out and based on
the alleged effects of the
compound/composition